# EUROPEAN PATENT SPECIFICATION

(11) **EP 1 428 890 B1**
(45) Date of publication and mention of the grant of the patent: **06.08.2008**
(21) Application number: 02762825.4
(22) Date of filing: 22.08.2002
(51) Int. Cl.: C12Q 1/68, C12N 15/09

(54) **METHOD OF JUDGING MISMATCH BETWEEN SINGLE-STRANDED NUCLEIC ACID MOLECULES**
VERFAHREN ZUR BEURTEILUNG EINER FEHLPAARUNG ZWISCHEN EINZELSTRÄNGIGEN NUKLEINSÄUREMOLEKÜLEN
PROCEDE DE DETERMINATION D'UN MESAPPARIEMENT ENTRE DES MOLECULES D'ACIDE NUCLEIQUE MONOBRIN

(30) Priority: 24.08.2001 JP 2001253789
(43) Date of publication of application: 16.06.2004
(73) Proprietor: NOF CORPORATION, Tokyo 150-6019 (JP)
(72) Inventor: MARUYAMA, Atsushi, c/o TOKYO INST. OF TECHNOLOGY, Yokohama-shi, Kanagawa 226-8503 (JP)
(74) Representative: Woods, Geoffrey Corlett
(86) International application number: PCT/JP2002/008448
(87) International publication number: WO 2003/018841

(56) References cited:
- EP-A- 1 391 519
- WO-A-02/48404
- GELFAND C.A. ET AL.: 'A quantitative method for evaluating the stabilities of nucleic acids' PROC. NATL. ACAD. SCI. USA vol. 96, no. 11, 1989, pages 6113 - 6118, XP002925481
- KIN ENSHO ET AL.: 'Polycation kyo jugotai no DNA-sa kokan sokushin koka' BIO KOBUNSHI SYMPOSIUM KOEN YOSHISHU vol. 11, July 2001, pages 41 - 42, XP002960293
- KIN ENSHO ET AL.: 'Cation-sei graft kyo jugotai ni yoru DNA-sa kokan han'no no kasoku' BIO KOBUNSHI SYMPOSIUM KOEN YOSHISHU vol. 10, 2000, pages 67 - 68, XP002960294
- KIM W.J. ET AL.: 'Comb-type cationic copolymer expedites DNA strand exchange while stabilizing DNA duplex' CHE. EUR. J. vol. 7, no. 1, January 2001, pages 176 - 180, XP002960295
- MANAMI UEDA ET AL.: 'Polycation kushigata kyo jugotai ni yoru sanjusa DNA no anteika kiko no kaiseki' POLYMER PREPRINTS, JAPAN vol. 50, no. 5, May 2001, page 990
- SHUJIRO SAKAKI ET AL.: 'MPC polymer ni yoru 1 enki takata (SNPs) no kokateki na ninshiki' POLYMER PREPRINTS, JAPAN vol. 50, no. 5, May 2001, page 992, XP002960296

## Description

### TECHNICAL FIELD

The present invention relates to a method for judging whether or not a mismatch occurs between a sample single-stranded nucleic acid molecule and a standard single-stranded nucleic acid molecule. The method can discriminate the occurrence of a sequence mismatch accurately even when the mismatch occurs only in a single base, and therefore can be utilized in DNA diagnoses and the like.

### BACKGROUND OF THE INVENTION

As the method for specifically detecting a nucleic acid molecule having a specific nucleotide sequence, hybridization technique has been widely employed. This technique has been utilized for the detection of a particular bacterium or virus and for the isolation of a particular gene. In recent years, the technique has been also utilized in fields including the so-called gene diagnoses in which a subtle mutation in human genomic DNA is detected to determine the susceptibility to a disease or response to a drug in an individual. The difference in susceptibility to a disease or response to a drug is often contributed to the difference of a single base in the genomic DNA. Therefore, in the gene diagnoses, it is needed to adjust the conditions so that a DNA used as a probe can hybridize only to a nucleic acid molecule that is entirely complementary to the DNA but does not hybridize to a nucleic acid molecule that has any mismatch. To achieve such a stringent hybridization, heretofore, the melting temperatures (Tₘ) for a double-stranded nucleic acid molecule having an entire complementarity and a double-stranded nucleic acid molecule having a mismatch are predicted previously, and the temperatures and salt concentrations for the hybridization are adjusted based on the predicted melting temperatures.

In the case where a nucleic acid molecule to be detected is shorter in length, hybridization with retaining specificity to some extent can be achieved by the adjustment of temperatures and the like as stated above. In the case where the nucleic acid molecule to be detected is longer in length, however, the difference in melting temperature between a double-stranded nucleic acid molecule having an entire complementarity and a double-stranded nucleic acid molecule having any mismatch is small, and therefore it is difficult to achieve hybridization in which the two double-stranded nucleic acid molecules can be distinguished from each other.

On the other hand, it is known that a graft copolymer having a cationic polymer main chain with hydrophilic polymer side chains can stabilize a nucleic acid molecule (Japanese Patent Application Publication Nos. 10-45630 and 10-158196) and can accelerate the exchange reaction between nucleic acid molecules (Japanese Patent Application Publication No. 2001-78769). However, nothing is known about the use of the graft copolymer for the purpose of eliminating the hybridization to a nucleic acid molecule having a mismatch as stated above.

WO 02/48404 describes a method for forming a duplex from a polynucleotide probe and a target nucleic acid, by providing the probe to the test sample under conditions permitting hybridization and also providing a synthetic polycationic polymer to increase the association rate of the probe and the target nucleic acid.

EP-A-1 391 519 describes an inhibitor for use in increasing accuracy of a hybridization reaction. Also described is a method comprising contacting a sample with a test agent capable of hybridizing with a target nucleic acid, in the presence of the inhibitor and under conditions suitable for hybridization, and detecting any hybridization that occurs.

As mentioned above, in genetic diagnoses, information about whether or not a particular nucleic acid molecule is entirely complementary to a probe without the occurrence of even a single base mismatch is of extreme importance.

The present invention is accomplished in these technical situations. The object of the present invention is to provide a means for judging accurately whether or not a sequence mismatch or mismatches occur between a single-stranded nucleic acid molecule employed as a sample and a probe.

### DISCLOSURE OF THE INVENTION

The present inventors have made extensive studies for the purpose of solving the problems stated above. As a result, the inventors have found that when a double-stranded DNA is allowed to co-exist with a complementary single-stranded DNA of one strand of the double-stranded DNA in the presence of a cationic polymer such as a graft polymer as stated above, large differences are produced in the rate and ratio of substitution between the DNAs by the occurrence of a mismatch in the complementary single-stranded DNA. Based on this finding, the invention has been accomplished.

That is, the present invention provides a method for judging whether or not a sequence mismatch or mismatches occur between a sample single-stranded nucleic acid molecule and a standard single-stranded nucleic acid molecule, the method comprising the steps of: (a) allowing a double-stranded nucleic acid molecule consisting of the standard single-stranded nucleic acid molecule and a complementary strand thereof to co-exist with the sample single-stranded nucleic acid molecule in the presence of a cationic polymer; and (b) determining the rate or ratio of the substitution of the complementary strand of the standard single-stranded nucleic acid molecule by the sample single-stranded nucleic acid molecule.

The present invention also provide a method for judging whether or not a sequence mismatch or mismatches occur between a sample single-stranded nucleic acid molecule and a standard single-stranded nucleic acid molecule, the method comprising the steps of: (a) allowing the standard single-stranded nucleic acid molecule to contact with the sample single-stranded nucleic acid molecule to form a double-stranded nucleic acid molecule; (b) allowing the double-stranded nucleic acid molecule formed in step (a) to co-exist with an entirely complementary strand of the standard single-stranded nucleic acid molecule in the presence of a cationic polymer; and (c) determining the rate or ratio of the substitution of the sample single-stranded nucleic acid molecule by the entirely complementary strand of the standard single-stranded nucleic acid molecule.

The present invention further provides a method for judging whether of not a sequence mismatch or mismatches occur between a sample single-stranded nucleic acid molecule and a standard single-stranded nucleic acid molecule, the method comprising the steps of: (a) allowing the standard single-stranded nucleic acid molecule to contact with an entirely complementary strand thereof and the sample single-stranded nucleic acid molecule in the presence of a cationic polymer; and (b) determining the formation ratio between a double-stranded nucleic acid molecule formed from the standard single-stranded nucleic acid molecule and the entirely complementary strand thereof and a double-stranded nucleic acid molecule formed from the standard single-stranded nucleic acid molecule and the sample single-stranded nucleic acid molecule.

Hereinbelow, the present invention will be described in detail.

The present invention provides a method for judging whether or not a sequence mismatch or mismatches occur between a sample single-stranded nucleic acid molecule (i.e., a single-stranded nucleic acid molecule employed as a sample) and a standard single-stranded nucleic acid molecule (i.e., a single-stranded nucleic acid molecule employed as a standard) using a cationic polymer.

The cationic polymer may be any one, as long as it can produce a difference in rate or ratio of substitution as state above at a detectable level. Examples include: (A) a graft copolymer having a main chain made up of a polymer composed of a monomer capable of forming a cationic group and side chains made up of a hydrophilic polymer; and (B) a polymer formed by the polymerization of a monomer having a phosphorylcholine-like group and a cationic monomer having a cationic group (hereinbelow, abbreviated to "PC polymer") as described in detail below.

### (A) Graft copolymer having a main chain made up of a polymer composed of a monomer capable of forming a cationic group and side chains made up of a hydrophilic polymer

In this graft copolymer, the monomer capable of forming a cationic group includes, for example, amino acids such as lysine, arginine and histidine; saccharides such as glucosamine; and synthetic monomers such as allyl amines, ethylene imine, diethylaminoethyl methacrylate and dimethylaminoethyl methacrylate. The polymer composed of a monomer capable of forming a cationic group includes, for example, polylysine or poly(ally amine). The hydrophilic polymer includes, for example, water-soluble polyalkylene glycols such as polyethylene glycol; water-soluble polysaccharides such as dextran, pullulan, amylose and arabinogalactan; water-soluble poly(amino acids) containing hydrophilic amino acids such as serine, asparagine, glutamine and threonine; water-soluble polymers synthesized using acrylamide or a derivative thereof as a monomer; water-soluble polymers synthesized using methacrylic acid, acrylic acid or a derivative thereof (e.g., hydroxyethyl methacrylate) as a monomer; and polyvinyl alcohol or a derivative thereof. More preferred cationic polymers are, for example, α-poly(L-lysine)-graft-dextran (hereinafter, abbreviated to "α-PLL-g-Dex"), ω-poly(L-lysine)-graft-dextran (hereinafter, abbreviated to "ω-PLL-g-Dex") and poly(allyl amine)-graft-dextran (hereinafter, abbreviated to "PAA-g-Dex") represented by the following formulae, respectively, all of which are described in Bioconjugate Chem., 9, 292-299 (1998). α-PLL-g-Dex:

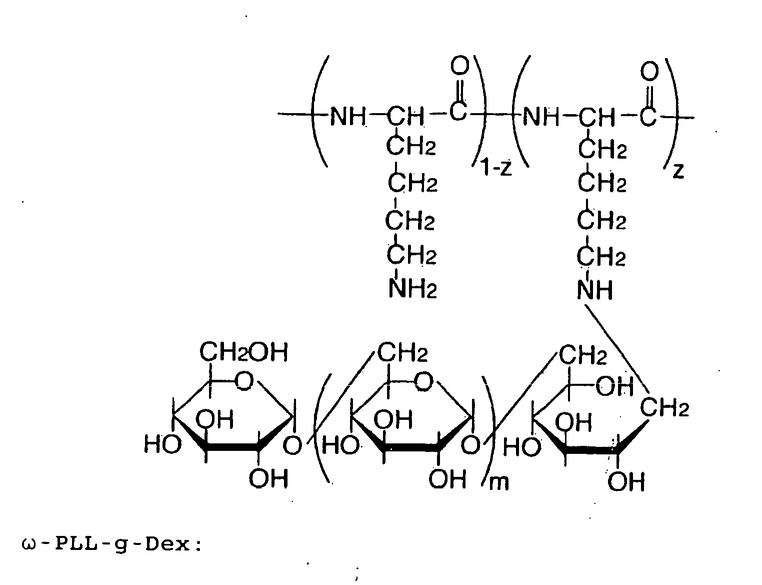

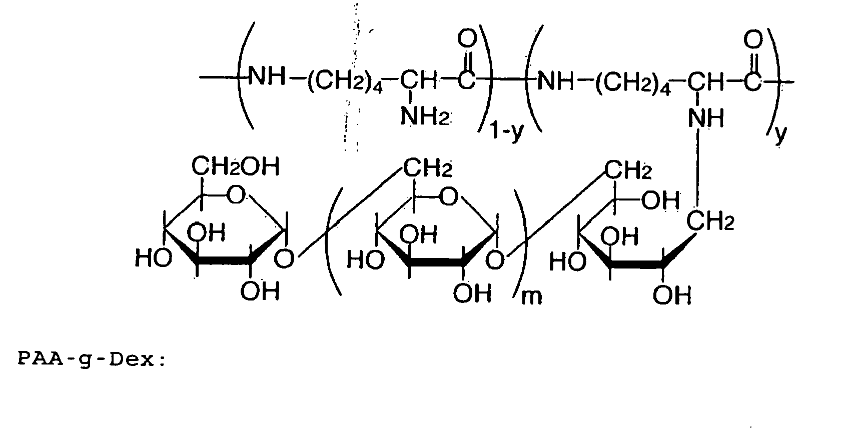

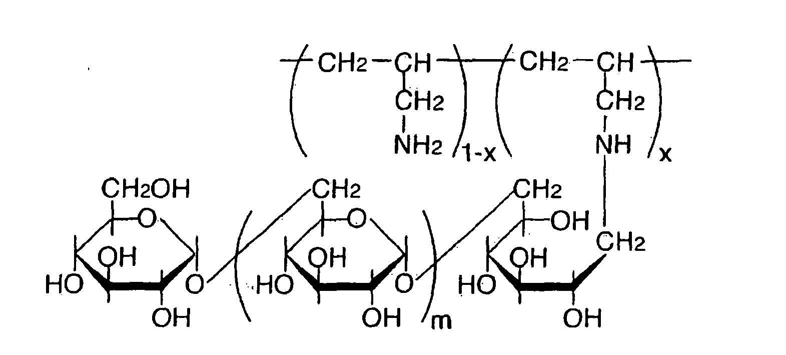

The molecular weight, the lengths of the side chain and the main chain, the degree of grafting and the like of the cationic polymer are not particularly limited, and may be defined depending on the specific intended use. The cationic polymer can be produced according to any of the known methods (e.g., the method described in Japanese Patent Application Publication No. 10-45630).

### (B) PC polymer:

In this polymer, the monomer having a phosphorylcholine-like group (hereinafter, abbreviated to "PC monomer") is represented by formula (I) below: 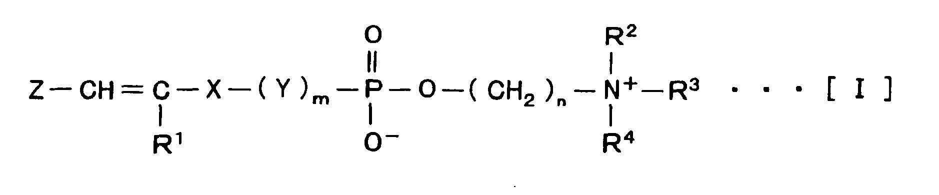 wherein, X denotes a divalent organic residue; Y denotes an alkyleneoxy group having 1 to 6 carbon atoms; Z denotes a hydrogen atom or R⁵-O-(C=O)- wherein R⁵ denotes an alkyl group having 1 to 10 carbon atoms or a hydroxyalkyl group having 1 to 10 carbon atoms; R¹ denotes a hydrogen atom or a methyl group; each of R², R³ and R⁴ independently denotes a hydrogen atom or an alkyl or hydroxyalkyl group having 1 to 6 carbon atoms; m is 0 or 1; and n in an integer of 1 to 4.

The divalent organic residue X in formula (I) includes, for example, -C₆H₄-, -C₆H₁₀-, -(C=O)-O-, -O-, -CH₂-O-, - (C=O)NH-, -O-(C=O)-, -O-(C=O)-O-, -C₆H₄-O-, -C₆H₄-CH₂-O-, and -C₆H₄- (C=O)-O-.

The Y in formula (I) is an alkyleneoxy group having 1 to 6 carbon atoms, such as methyloxy, ethyloxy, propyloxy, butyloxy, pentyloxy and hexyloxy groups.

The Z in formula (I) denotes a hydrogen atom or a residue R⁵-O-(C=O)- wherein R⁵ is an alkyl group having 1 to 10 carbon atoms or a hydroxyalkyl group having 1 to 10 carbon atoms.

Here, the alkyl group having 1 to 10 carbon atoms includes, for example, methyl, ethyl, propyl, butyl, pentyl, hexyl, heptyl, octyl, nonyl and decyl groups.

The hydroxyalkyl group having 1 to 10 carbon atoms includes, for example, hydroxymethyl, 2-hydroxyethyl, 3-hydroxypropyl, 2-hydroxypropyl, 4-hydroxybutyl, 2-hydroxybutyl, 5-hydroxypentyl, 2-hydroxypentyl, 6-hydroxyhexyl, 2-hydroxyhexyl, 7-hydroxyheptyl, 2-hydroxyheptyl, 8-hydroxyoctyl, 2-hydroxyoctyl, 9-hydroxynonyl, 2-hydroxynonyl, 10-hydroxydecyl and 2-hydroxydecyl groups.

The PC monomer includes, for example, 2-((meth)acryloyloxy)ethyl-2'-(trimethylammonio)ethyl phosphate, 3- ((meth) acryloyloxy) propyl-2'-(trimethylammonio)ethyl phosphate, 4-((meth)acryloyloxy)butyl-2'-(trimethylammonio)ethyl phosphate, 5- ((meth) acryloyloxy) pentyl-2'-(trimethylammonio)ethyl phosphate, 6-((meth)acryloyloxy)hexyl-2'-(trimethylammonio)ethyl phosphate, 2-((meth)acryloyloxy)ethyl-2'-(triethylammonio)ethyl phosphate, 2-((meth)acryloyloxy)ethyl-2'-(tripropylammonio)ethyl phosphate, 2-((meth)acryloyloxy)ethyl-2'-(tributylammonio)ethyl phosphate, 2-((meth)acryloyloxy)ethyl-2'-(tricyclohexylammonio)ethyl phosphate, 2-((meth)acryloyloxy)ethyl-2'-(triphenylammonio)ethyl phosphate, 2-((meth)acryloyloxy)ethyl-2'-(trimethanolammonio)ethyl phosphate, 2-((meth)acryloyloxy)propyl-2'-(trimethylammonio)ethyl phosphate, 2-((meth)acryloyloxy)butyl-2'-(trimethylammonio)ethyl phosphate, 2-((meth)acryloyloxy)pentyl-2'-(trimethylammonio)ethyl phosphate, 2-((meth)acryloyloxy)hexyl-2'-(trimethylammonio)ethyl phosphate, 2-(vinyloxy)ethyl-2'-(trimethylammonio)ethyl phosphate, 2-(allyloxy)ethyl-2'-(trimethylammonio)ethyl phosphate, 2-(p-vinylbenzyloxy)ethyl-2'-(trimethylammonio)ethyl phosphate, 2-(p-vinylbenzolyoxy)ethyl-2'-(trimethylammonio)ethyl phosphate, 2-(stylyloxy)ethyl-2'-(trimethylammonio)ethyl phosphate, 2-(p-vinylbenzyl)ethyl-2'-(trimethylammonio)ethyl phosphate, 2-(vinyloxycarbonyl)ethyl-2'-(trimethylammonio)ethyl phosphate, 2-(allyloxycarbonyl)ethyl-2'-(trimethylammonio)ethyl phosphate, 2-(acryloylamino)ethyl-2'-(trimethylammonio)ethyl phosphate, 2-(vinylcarbonylamino)ethyl-2'-(trimethylammonio)ethyl phosphate, ethyl-(2'-trimethylammonioethylphosphorylethyl) fumarate, butyl-(2'-trimethylammonioethylphosphorylethyl) fumarate, hydroxyethyl - (2'-trimethylammonioethylphosphorylethyl) fumarate, ethyl-(2'-trimethylammonioethylphosphorylethyl) maleate, butyl-(2'-trimethylammonioethylphosphorylethyl) maleate and hydroxyethyl-(2'-trimethylammonioethylphosphorylethyl) maleate.

Among these, 2-((meth)acryloyloxy)ethyl-2'-(trimethylammonio)ethyl phosphate is preferred, and 2-(methacryloyloxy)ethyl-2'-(trimethylammonio)ethyl phosphate (also termed "2-(methacryloyloxy)ethyl phosphorylcholine", hereinafter abbreviated to "MPC") is more preferred in terms of availability or the like.

The PC monomer can be produced according to any of the known methods. For example, it can be produced according to the known method described in Japanese Patent Application Publication No. 54-63025, 58-154591 or the like.

The cationic monomer having a cationic group used in the present invention includes, for example, a monomer having an primary amino group, a monomer having a secondary amino group, a monomer having a tertiary amino group or a monomer having a quaternary ammonium group. The monomer having a primary amino group includes, for example, allylamine (hydrochloride), aminoethyl (meth)acrylate (hydrochloride), 2-methylallyamine and 4-aminostylene. The monomer having a secondary amino group, a tertiary amino group or a quaternary ammonium group includes, for example, (meth)acrylamide, N-[3-(dimethylamino)propyl]methacrylamide (hydrochloride), N-[3-(dimethylamino)propyl]acrylamide (hydrochloride), 2-(dimethylamino) ethylmethacrylate (hydrochloride), 2-(dimethylamino) ethylacrylate (hydrochloride), [3-(methacryloyloxyamino)propyl]trimethylammonium chloride, [3-(acryloyloxyamino)propyl]trimethylannmonium chloride, [2-(methacryloyloxyamino)ethyl]trimethylannmonium chloride, [2-(acryloyloxyamino)ethyl]trimethylammonium chloride, 2-hydroxy-3-methacryloyloxypropyltrimethylammonium chloride (hereinafter, abbreviated to "QA") and 2-hydroxy-3-acryloyloxypropyltrimethylammonium chloride.

In particular, the combination of the PC monomer and the cationic monomer is preferably the combination of MPC and aminoethylacrylate (hydrochloride) which has a primary amino group or the combination of MPC and QA which has a quaternary ammonium group.

As used herein, the term "single-stranded nucleic acid molecule" primarily refers to a single-stranded DNA, but may also include RNA, nucleic acid-analogue molecules (e.g., PNA) and the like. The length of the sample single-stranded nucleic acid molecule is not particularly limited. However, it is preferred to use a nucleic acid molecule having a length of 12 bases or longer as a sample, with which the occurrence of a single-base mismatch is hardly discriminated.

The judgment method of the present invention may be one of the following first to third methods.

### (1) First method

The first method comprises the steps (a) and (b) below.

In the step (a), a double-stranded nucleic acid molecule consisting of the standard single-stranded nucleic acid molecule and a complementary strand thereof is allowed to co-exist with the sample single-stranded nucleic acid molecule in the presence of a cationic polymer. When there is no sequence mismatch between the sample single-stranded nucleic acid molecule and the standard single-stranded nucleic acid molecule, the complementary strand of the standard single-stranded nucleic acid molecule is substituted by the sample single-stranded nucleic acid molecule at a high rate and with a high ratio. In contrast, when there is any sequence mismatch between them, the complementary strand is rarely substituted by the sample single-stranded nucleic acid molecule.

The amount of the cationic polymer used is not particularly limited, but is preferably such an amount that the ratio of the cationic groups in the cationic polymer to the phosphate groups in the nucleic acids ranges from 0.1 to 1,000. The time period for which the double-stranded nucleic acid molecule and the sample single-stranded nucleic acid molecule are allowed to co-exist together is not also particularly limited, but is preferably from about 1 minute to about 16 hours.

The single-stranded nucleic acid molecule which can form a double strand with the standard single-stranded nucleic acid molecule may not be an entirely complementary strand and may have one to several mismatches in the sequence. In this case, if the sample single-stranded nucleic acid molecule is entirely complementary to the standard single-stranded nucleic acid molecule, the rate and ratio of substitution between the two molecules become higher.

In the step (b), the rate or ratio of the substitution of the complementary strand of the standard single-stranded nucleic acid molecule by the sample single-stranded nucleic acid molecule is determined. As stated above, when there is no mismatch, the rate and ratio of the substitution of the complementary strand of the standard single-stranded nucleic acid molecule by the sample single-stranded nucleic acid molecule are remarkably higher than those obtained when there is any mismatch. Thus, the determination of the rate or ratio of substitution enables to judge whether or not a mismatch occurs between the two single-stranded nucleic acid molecules. If there is no mismatch, the rate of substitution is about 10 to 1,000 times greater than that obtained when there is any mismatch. Thus, when setting the reaction time and conditions appropriately, the value of the ratio of substitution is generally determined to be about 40 to 100% in the case where no mismatch is contained, while it is generally determined to be about 0 to 10% in the case where a single-base mismatch is contained.

For achieving more accurate judgment, it is preferred to perform a control experiment using an entirely complementary strand of the standard single-stranded nucleic acid molecule in place of the sample single-stranded nucleic acid molecule. In this case, if the rate and ratio of substitution of the sample single-stranded nucleic acid molecule is equivalent to those of the entirely complementary strand, then it can be concluded that there is no mismatch in the sequences.

The step (b) may be performed simultaneously with the step (a), which is rather preferred for the determination of the rate of substitution.

The method for determination of the rate and ratio of substitution is not particularly limited, and the rate or ratio of substitution can be determined with an enzyme, fluorescent substance or luminescent substance. In particular, use of fluorescence resonance energy transfer (FRET) method is preferred for such determination. In this method, the standard single-stranded nucleic acid molecule is labeled with a donor fluorescent dye (e.g., fluorescein isothiocyanate) and a complementary strand thereof is labeled with an acceptor fluorescence dye (e.g., tetramethyl rhodamine). In the state where a double strand is formed between the nucleic acid molecule labeled with the donor fluorescent dye and the nucleic acid molecule labeled with the acceptor fluorescent dye, the donor fluorescent dye emits no fluorescent light. In contrast, once the nucleic acid molecule labeled with the acceptor fluorescent dye is substituted by the sample single-stranded nucleic acid molecule, the donor fluorescent dye comes to emit fluorescent light. The rate and ratio of substitution thus can be determined indirectly by measuring the fluorescence intensity of the donor fluorescent dye. The measurement may be made sequentially during or after the substitution reaction by means of electrophoresis, such as capillary electrophoresis, polyacrylamide gel electrophoresis (PAGE) and agarose gel electrophoresis (AGE). Alternatively, multiple samples may be measured simultaneously using a titer plate, such as a 96-, 384- or 1536-well plate.

### (2) Second method

The second method comprises the steps (a), (b) and (c) below.

In the step (a), the standard single-stranded nucleic acid molecule is allowed to contact with the sample single-stranded nucleic acid molecule to form a double-stranded nucleic acid molecule. The formation of the double-stranded nucleic acid molecule can be performed in the same manner as in the conventional hybridization method. The standard single-stranded nucleic acid molecule may be immobilized on a substrate.

In the step (b), the double-stranded nucleic acid molecule formed in the step (a) is allowed to co-exist with an entirely complementary strand of the standard single-stranded nucleic acid molecule in the presence of a cationic polymer. In the case where there is any sequence mismatch between the sample single-stranded nucleic acid molecule and the standard single-stranded nucleic acid molecule, the sample single-stranded nucleic acid molecule is substituted by the entirely complementary strand of the standard single-stranded nucleic acid molecule at a very high rate and with a very high ratio. In contrast, in the case where there is no mismatch, although the substitution may also occur, the rate and ratio of the substitution are lower than those obtained in the case where there is any mismatch.

The amount of the cationic polymer used may be the same as that employed in the first method. The time period for which the double-stranded nucleic acid molecule and the entirely complementary strand of the standard single-stranded nucleic acid molecule are allowed to co-exist together may also be the same as that employed in the first method.

In the step (c), the rate or ratio of the substitution of the sample single-stranded nucleic acid molecule by the entirely complementary strand of the standard single-stranded nucleic acid molecule is determined. As stated above, in the case where any mismatch is contained, the rate and ratio of the substitution of the sample single-stranded nucleic acid molecule by the entire complementary strand of the standard single-stranded nucleic acid molecule are remarkably higher than those obtained in the case where no mismatch is contained. Thus, the determination of the rate or ratio of the substitution enables to judge whether or not a mismatch occurs between the two single-stranded nucleic acid molecules. When any mismatch is contained, the rate of substitution is generally about 10 to 1,000 times greater than that obtained when no mismatch is contained. Thus, when setting the reaction time and conditions appropriately, the value of the ratio of substitution is generally determined to be about 0 to 10% in the case where no mismatches is contained, while it is generally determined to be about 40 to 100% in the case where a single-base mismatch is contained.

For achieving more accurate judgment, as in the case of the first method, it is preferred to perform a control experiment using an entirely complementary strand of the standard single-stranded nucleic acid molecule in place of the sample single-stranded nucleic acid molecule.

The step (c) may be performed simultaneously with the step (b), which is rather preferred for the determination of the rate of substitution.

The method for determination of the ratio of substitution is preferably FERT as in the case of the first method, but is not particularly limited thereto.

### (3) Third method

The third method comprises the steps (a) and (b) below.

In the step (a), the standard single-stranded nucleic acid molecule is allowed to contact with an entirely complementary strand thereof and the sample single-stranded nucleic acid molecule. This results in the formation of a double strand between the standard single-stranded nucleic acid molecule and the entirely complementary strand thereof (a control double strand). In this time, the sample single-stranded nucleic acid molecule may also form a double strand with the standard single-stranded nucleic acid molecule (a sample double strand), if there is a certain degree of complementary between them. In the case where there is no mismatch between the sample single-stranded nucleic acid molecule and the standard single-stranded nucleic acid molecule, the control double strand and the sample double strand are formed almost in equivalent quantities. In contrast, in the case where there is any sequence mismatch, the control double strand is formed in a larger quantity than the sample double strand.

The formation of the double-stranded nucleic acid molecules can be achieved in the same manner as in the conventional hybridization method. The standard single-stranded nucleic acid molecule may be immobilized on a substrate.

The amount of the cationic polymer used may be the same as that employed in the first method.

In the step (b), the formation ratio between the double-stranded nucleic acid molecule formed from the standard single-stranded nucleic acid molecule and the complimentary strand thereof (control double strand) and the double-stranded nucleic acid molecule formed from the standard single-stranded nucleic acid molecule and the sample single-stranded nucleic acid molecule (sample double strand) is determined. As stated above, in the case where there is no sequence mismatch, the control double strand and the sample double strand are formed almost in equivalent quantities. In contrast, in the case where there is any mismatch, the control double strand is formed in a larger quantity than the sample double strand. Then, by the determination of the formation ratio between the control double strand and the sample double strand, it becomes possible to discriminate the occurrence of a sequence-sequence mismatch. The values of the formation ratio between the control double strand and the sample double strand may vary depending on various factors. However, when the same concentrations are employed for all of the nucleic acid molecules, the formation ratio is generally determined to be about 1:1 in the case where no mismatch is contained, while it is generally determined to be about 1.5-5:1 in the case where a single-base mismatch is contained.

The step (b) may be performed simultaneously with the step (a), which is rather preferred for the determination of the formation rate of the double strands.

The method for determination of the formation ratio is preferably FERT as in the case of the first method, but is not particularly limited thereto.

### BRIEF DESCRIPTION OF DRAWINGS

Fig. 1 shows diagrams showing the melting curves of a double strand having an entirely complementarity and a double strand having a single base mutation, respectively.
Fig. 2 is a diagram showing the time course of the ratio of strand substitution between a double strand (F2/T2) and each of single strands (M2, M2misG, M2misA and M2misC).
Fig. 3 is a diagram showing the correlation between the presence of α-PLL-g-Dex and the ratio of substitution between a double strand (F2/T2) and each of single strands (M2 and M2misG).

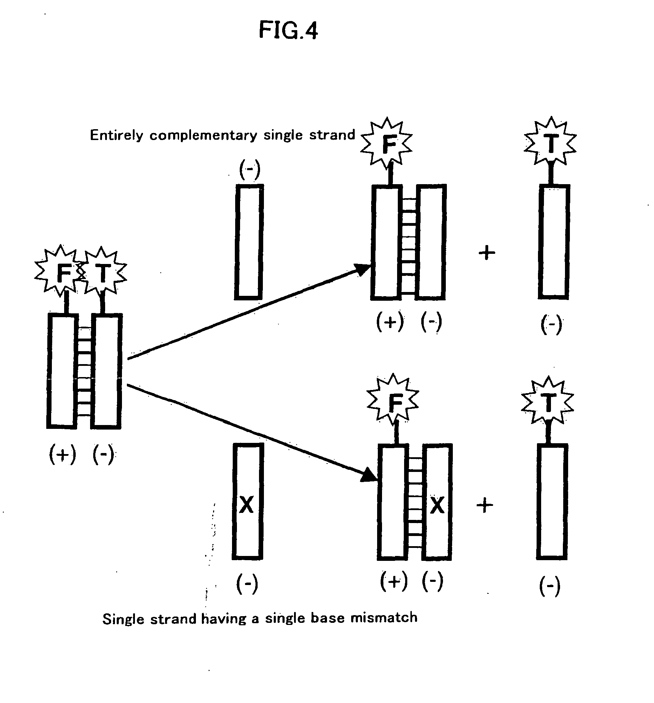

Fig. 4 is a diagram showing the schematic of the strand exchange experiment between a double strand and a single strand.

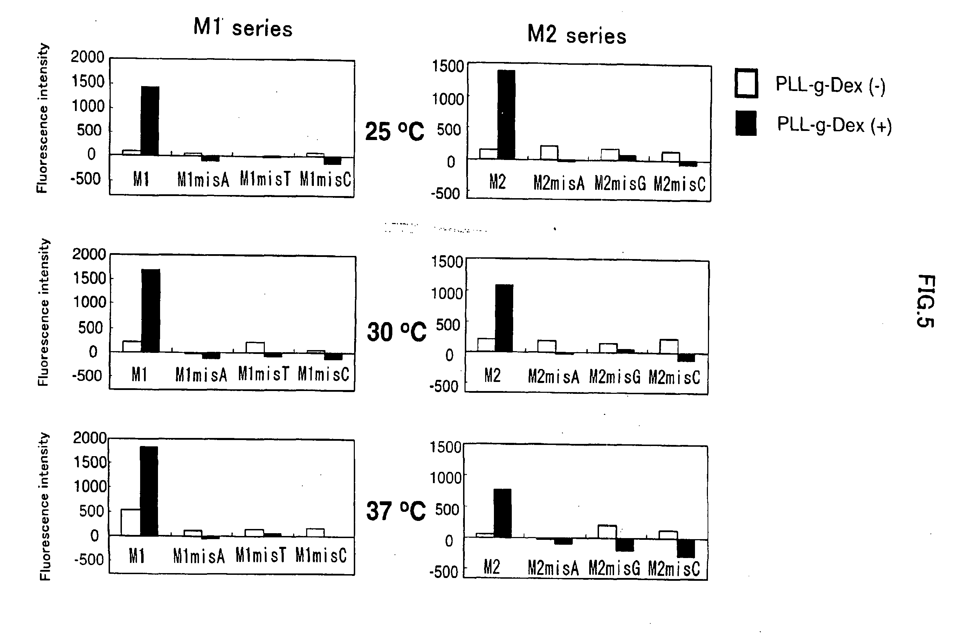

Fig. 5 shows diagrams showing the correlation between the temperature/state of mutation of single strand and the ratio of substitution between a double strand and a single strand.
Fig. 6 is a diagram showing the correlation between the presence of α-PLL-g-Dex and the ratio of substitution between a double strand (F2/T2) and each of single strands (M50 and M50mis1).
Fig. 7 is a diagram showing the time course of the ratio of strand substitution between a double strand (F3/T3) and each of single strands (M3 and M3miss1)

### BEST MODE FOR CARRYING OUT THE INVENTION

### [Synthesis Example 1] Synthesis of PC polymer 1

A polymerization tube was charged with 1.1 g of MPC and 0.6 g of 2-aminoethyl methacrylate hydrochloride (hereinbelow, abbreviated to "AEMA") as monomers, 0.4 g of 2,2-azobis(2-methylpropionamidine) dihydrochloride (Wako Pure Chemical Industries, Ltd.; hereinbelow, abbreviated to "V-50") as an initiator and 12.9 g of distilled water as a polymerization solvent, and then dissolved together homogeneously. The solution was purged with argon for 10 minutes and the tube was then sealed. After the sealing was completed, the polymerization reaction was performed at 60°C for 8 hours. After the polymerization was completed, the reaction solution was cooled to room temperature, the sealed polymerization tube was opened, and the solution was placed in a dialysis membrane (trade name: "Spectrum/por.membrans Mw Co, 6000-8000", Spectrum Medical Industries, Inc.) to dialyze the polymerization solution with distilled water in a volume 10-times the volume of the polymerization solution. The replacement of distilled water once daily was continued for 7 days to remove the unreacted monomers and the initiator. The solution was freeze-dried to give 1.5 g of PC polymer 1.

Twenty mg of the powder obtained above was dissolved in 1.5 mL of heavy water (D₂O). The resulting heavy water solution was subjected to ¹H-NMR analysis using JNM-EX270 (Japan Electron Optic Laboratory Ltd.) to determine the molar ratio between the PC polymer and the cationic monomer.

Ten mg of the PC polymer 1 was dissolved in 1.0 mL of Dulbecco's phosphate buffer (hereinbelow, abbreviated to "DPBS"). To this solution was added 1.0 mg/mL of propionic acid succinimide ester (Wako Pure Chemical Industries, Ltd.) which had been dissolved in dimethylformamide (hereinbelow, abbreviated to "DMF"). The solution was incubated at room temperature for 3 hours. After the incubation was completed, the solution was placed in a dialysis membrane (trade name: "Spectrum/por.membrans Mw Co, 6000-8000", Spectrum Medical Industries, Inc.) to dialyze the polymerization solution with distilled water in a volume 10-times the volume of the polymerization solution. The replacement of distilled water once daily was continued for 7 days. The solution was freeze-dried to give 8.5 mg of propylated PC polymer 1. The propylated polymer powder was dissolved in chloroform:methanol = 6:4 (v/v) containing 0.5 wt% of lithium chloride to prepare a 0.5 wt% polymer solution. The solution was filtrated through a 45 µm membrane filter to give a test solution.

GPC analysis was performed on MIXED-C (two columns) (Polymer Laboratories, Ltd.), using chloroform:methanol = 6:4 (v/v) containing 0.5 wt% of lithium chloride as an elution solvent; polymethyl methacrylate (Polymer Laboratories, Ltd.) as a standard; a parallax reflectometer for the detection; and a molecular weight calculation program (GPC program suited for SC-8020) included in an integrator (Tosoh Corporation) for the determination of weight average molecular weight (Mw), number average molecular weight (Mn) and molecular weight distribution (Mw/Mn); at a flow rate of 1.0 mL/min., at a sample solution loading amount of 100 µL and at a column temperature of 40°C.

The analysis showed that the PC polymer 1 had a MPC/AEMA molar ratio of 65/35, Mn = 393,000, Mw = 515,000 and Mw/Mn = 1.3.

### [Synthesis Example 2] Synthesis of PC polymer 2

The same procedure as in Synthesis Example 1 was performed, except that 0.8 g of a 50% aqueous solution of 2-hydroxy-3-methacryloyloxypropyltrimethylannmonium chloride (hereinbelow, abbreviated to "QA") was used in place of AEMA, V50 was used in an amount of 0.12 g and distilled water was used in an amount of 6.0 g, thereby giving 1.4 g of PC polymer 2.

Twenty mg of the powder obtained above was dissolved in 1.5 mL of heavy water (D₂O). The resulting heavy water solution was subjected to ¹H-NMR analysis using JNM-EX270 (Japan Electron Optic Laboratory Ltd.) to determine the molar ratio between the PC polymer and the cationic monomer.

The resultant aqueous copolymer solution was diluted with distilled water to 0.5 wt%, and the solution was filtrated through a 45 µm membrane filter to give a test solution.

GPC analysis was performed on G3000PWx1 (two columns) (Tosoh Corporation), using distilled water as an elution solvent; polyethylene glycol (Polymer Laboratories, Ltd.) as a standard; a parallax reflectometer for the detection; and a molecular weight calculation program (GPC program suited for SC-8020) included in an integrator (Tosoh Corporation) for the determination of weight average molecular weight (Mw), number average molecular weight (Mn) and molecular weight distribution (Mw/Mn); at a flow rate of 1.0 mL/min., at a sample solution loading amount of 100 µL, and at a column temperature of 40°C.

The analysis showed that the PC polymer 2 had a MPC/QA molar ratio of 70/30, Mn = 295,000, Mw = 389,000 and Mw/Mn = 1.3.

### [Example 1] Preparation of oligodeoxynucleotides (ODNs) and double-stranded sequences

The ODNs shown in Table 1 were purchased from Nippn TechnoCluster, Inc. and were purified by reverse phase chromatography. Double-stranded DNAs were obtained by mixing strands which are complementary to each other in equal molar amounts in TE buffer, heating to 90°C and then cooling.

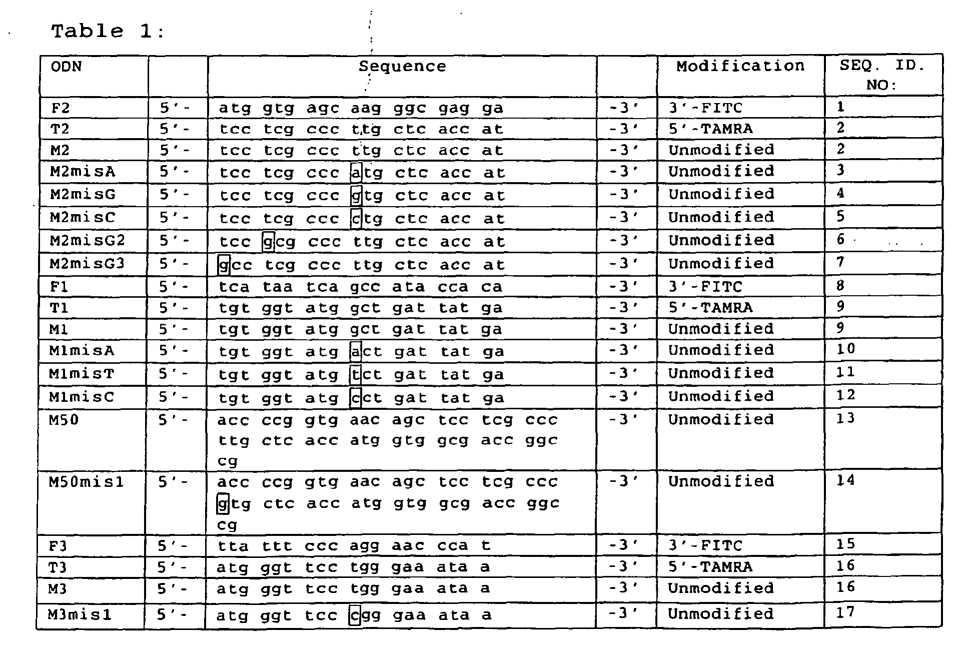

Each of the ODAs will be explained below briefly.

### (1) M2 series

F2 and T2 are 20-base sequences which are entirely complementary to each other, and are labeled with fluorescein isothiocyanate (FITC) and tetramethyl rhodamine (TAMRA) at the 3' end and at the 5' end, respectively. M2 is an unlabeled sequence corresponding to T2. M2misG, M2misA and M2misC are sequences each having a single base mutation at the center of M2. M2misG2 and M2misG3 are sequences having a single base mutation at the fourth base and the first base from the 5' end of M2, respectively.

### (2) M1 series

F1 and T1 are 20-base sequences which are entirely complementary to each other, and are labeled with FITC and TAMRA at the 3' end and at the 5' end, respectively. M1 is an unlabeled sequence corresponding to T1. M1misA, M1misT and M1misC are sequences each having a single base mutation at the center of M1.

### (3) M50 series

M50 is a 50-base sequence having a sequence complementary to T2 at the center. M50mis1 is a sequence having a single base mutation at the center of M50.

### (4) M3 series

F3 and T3 are 19-base sequences which are entirely complementary to each other, and are labeled with FITC and TAMRA at the 3' end and at the 5' end, respectively. M3 is an unlabeled sequence corresponding to T3. M3misl is a sequence having a single base mutation at the center of M3.

### [Example 2] Measurement of melting temperature

An entirely complementary double strand and double strands having a single base mutation were formed from F2 and each of M2, M2misG, M2misA and M2misC. Each of the double strands was diluted in 10 mM sodium phosphate buffer (PBS, pH 7.2) containing 150 mM NaCl to a concentration of 0.83 µM, and then was subjected the measurement of a melting temperature on Beckman UV/visible spectrophotometer equipped with a Micro Tm Analysis system. The measurement was made within the temperature range from 30 to 110°C at a temperature ramp rate of 1°C/minute. PLL-g-Dex (α-PLL-g-Dex) was added at a charge ratio (the amount of amino groups in PLL-g-Dex relative to the amount of phosphate groups in the DNA molecules) of 2, and then the melting temperature was measured in the same manner.

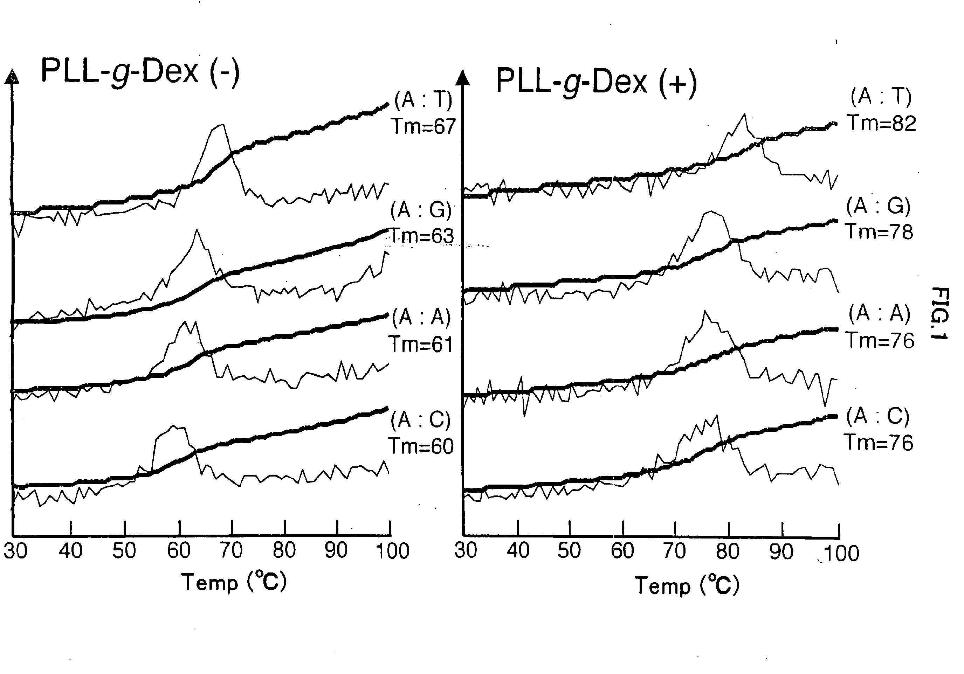

The results are shown in Fig. 1. Each thick line represents a melting curve, and each thin line represents a first order differentiation curve thereof. The melting of the double strands is observed at 50-70°C in the absence of PLL-g-Dex and at 70-90°C in the presence of PLL-g-Dex. The melting temperatures under different conditions are summarized in Table 2.

**Table 2: Melting temperature of each double-stranded sequence**

| Mutation | In the absence of PLL-g-Dex | In the presence of PLL-g-Dex |
|---|---|---|
| None (F2/M2) | 67°C | 82°C |
| T **->** G (F2/M2misG) | 63°C | 78°C |
| T **->** A (F2/M2misA) | 61°C | 76°C |
| T **->** C (F2/M2misC) | 60°C | 75°C |

Regardless the presence of PLL-g-Dex, all of the melting temperatures measured were close to one another and the maximum of the differences among them was 7°C. The ranges of melting temperature shift for all sequenced are overlapped one another. Thus, it is demonstrated that it is difficult to precisely distinguish these sequences from one another by the melting temperature measurement or the conventional hybridization method.

### [Example 3] Strand exchange between entirely complementary double strand and single strand (Part 1)

The effect of a single mutated base on the strand exchange between an entirely complementary strand and a single strand was examined. A double strand prepared with F2 and T2 was dissolved in PBS at a concentration of 12 nM, and then PPL-g-Dex was added thereto at a charge ratio of 3.3. Each of M2, M2misG, M2misA and M2misC was added as a single strand to the solution at a concentration of 12 nM while maintaining the solution at 37°C to initiate the strand exchange. The progress of the strand exchange was detected based on the recovery of the fluorescence of FITC (excitation wavelength: 490 nm, fluorescence wavelength: 520 nm) which had been quenched by TAMRA.

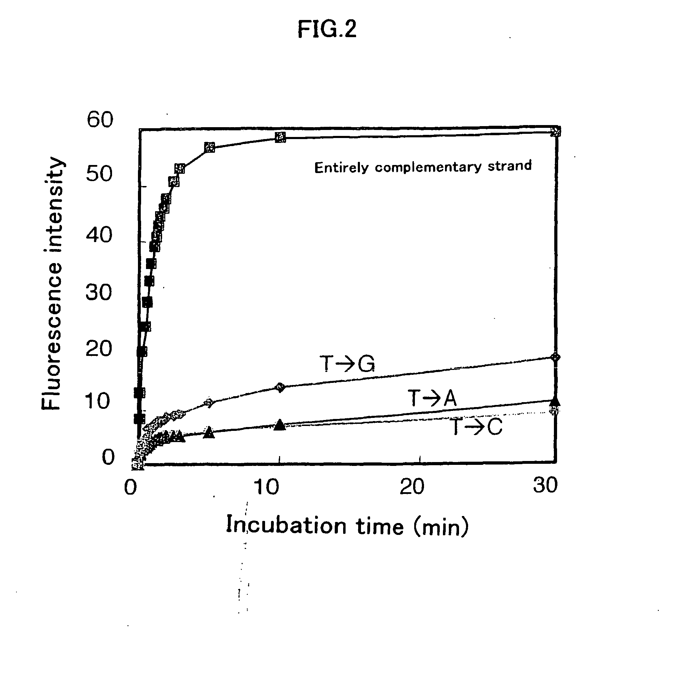

The results are shown in Fig. 2. It is shown that the strand exchange with the entirely complementary double strand proceeds rapidly, but, in contrast, the strand exchange with the single strand having a mutation proceeds slowly. The rate constant for strand exchange was calculated for each strand and is shown as a relative value in Table 3.

**Table 3**

| Mutation | Rate constant for strand exchange |
|---|---|
| None | 100 |
| T -> G | 1.14 |
| T -> A | 0.57 |
| T -> C | 0.43 |

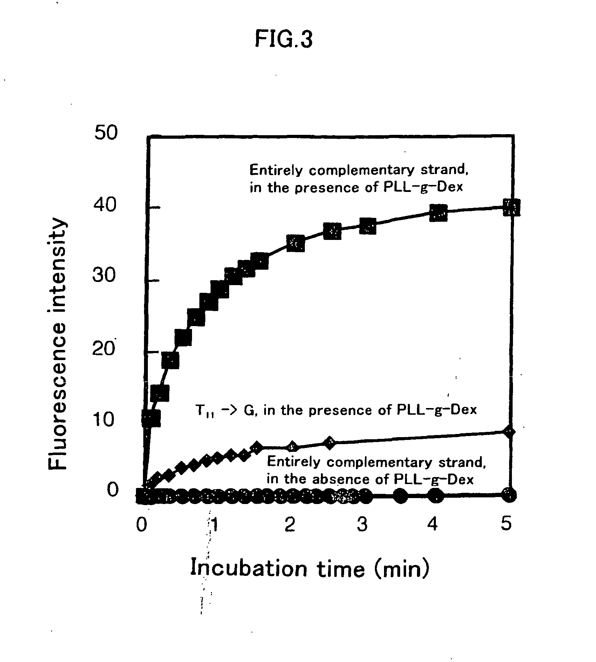

The rates of strand exchange with all of the strands having a mutation were about 1/100 relative to that with the entirely complementary strand. On the other hand, in the absence of PLL-g-Dex, strand exchange rarely occurred even with the entirely complementary strand (Fig. 3). It is found that strands having a mutation can be detected at a high sensitivity by the strand exchange accelerated by PLL-g-Dex.

### [Example 4] Strand exchange between entirely complementary double strand and single strand (Part 2)

The strand exchange was initiated in the same manner as in Example 3, and the fluorescence intensity was measured after 3 and 5 minutes. The results are shown in Table 4.

**Table 4: Fluorescence signal intensities after 3 and 5 minutes**

| Mutation | 3 min. | 5 min. |
|---|---|---|
| None (F2/M2) | 52.7 | 56.6 |
| T -> G (F2/M2misG) | 9.3 | 11.2 |
| T -> A (F2/M2misA) | 5.1 | 5.9 |
| T -> C (F2/M2misC) | 5.4 | 6.2 |

It is found that the reactions for 3 and 5 minutes can detect sequences having a mutation.

### [Example 5] Strand exchange between entirely complementary double strand and single strand (Part 3)

The strand exchange experiment was performed in the same manner as in Example 3, except that M2, M2misG, M2misG2 and M2misG3 were used as the single strands and PC polymers 1 and 2 were used in addition to PLL-g-Dex as the cationic polymers. The fluorescence intensity was measured under different conditions at 3 minutes after the experiment was started. The results are shown in Table 5.

**Table 5**

| Mutation | PLL-g-Dex | PC polymer 1 | PC polymer 2 |
|---|---|---|---|
| None (F2/M2) | 100 | 75.8 | 82.8 |
| T -> G (F2/M2misG) | 17.7 | 15.5 | 16.8 |
| T -> G (F2/M2misG2) | 51.9 | 49.8 | 50.2 |
| T -> G (F2/M2misG3) | 63.1 | 59.6 | 61.3 |

As can be seen from the table, it is found that the detection of sequences having a mutation can be achieved using the PC polymers as well as PLL-g-Dex. In addition, it is also found that it is possible to readily detect a single base mutation at the termini as well as a single base mutation at the center.

### [Example 6] Strand exchange between entirely complementary double strand and single strand (Part 4)

The strand exchange experiment was performed in the same manner as in Example 3, except for the following items, and the fluorescence intensity was measured under different conditions.
(1) The experiment was performed at three different temperatures: 25, 30 and 37°C.
(2) The exchange reaction was performed using a 96-well plate, and the measurement was made on a 96-well fluorescence plate reader (Wallac 1420 Multilabel counter, PerkinElmer Lifescience).
(3) In addition to the strand exchange experiment between a double strand formed from F2 and T2 and each of M2, M2misG, M2misA and M2misC, the strand exchange experiment between a double strand formed from F1 and T1 and each of M1, M1misA, M1misT and MmisC was also performed.

The results of the fluorescence intensity measurement are shown in Table 5.

### [Example 7] Strand exchange between entirely complementary double strand and single strand (Part 5)

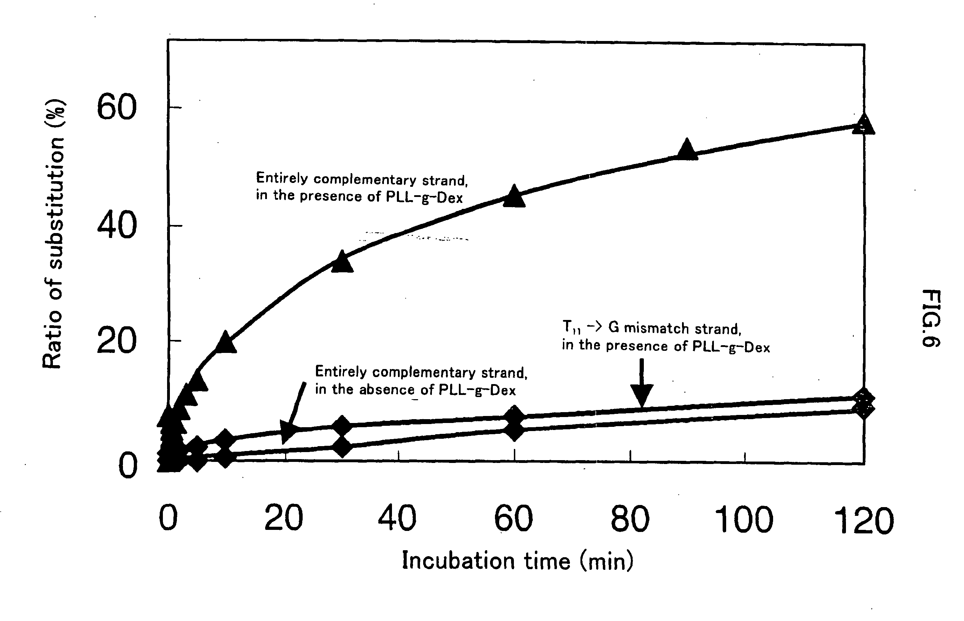

The strand exchange experiment was performed in the same manner as in Example 3, except that a charge ratio of 5 was employed and M50 and M50misl were used as single strands, thereby determining the time course of the change in substitution ratio between the double strand and each of the single strands. The results are shown in Fig. 6.

### [Example 8] Strand exchange between entirely complementary double strand and single strand (Part 6)

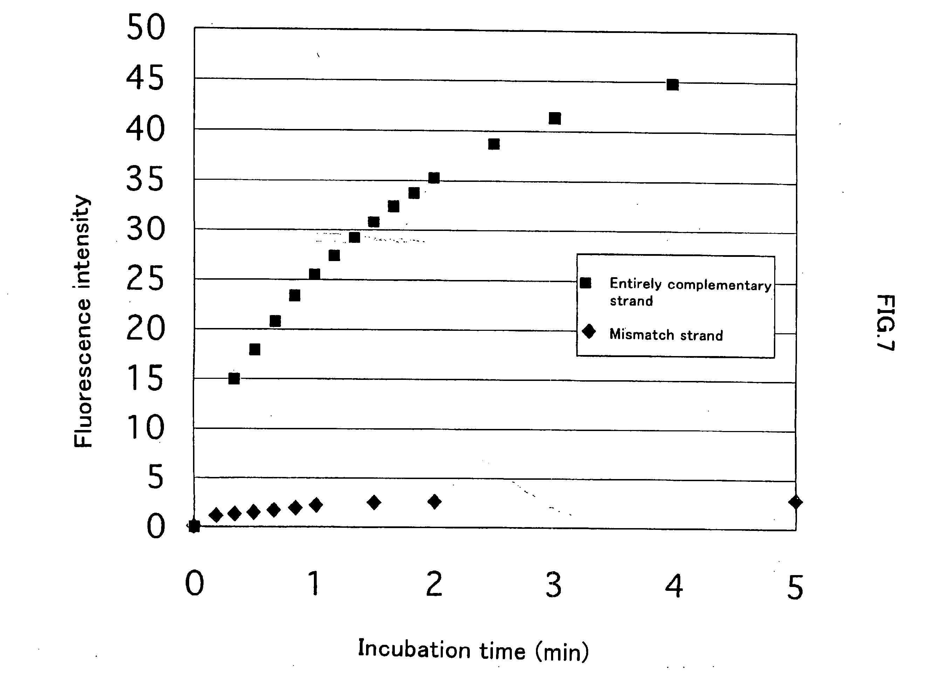

The strand exchange experiment was performed in the same manner as in Example 3, except that a charge ratio of 3 was employed, a double strand formed from F3 and T3 was used and M3 and M3mis1 were used as single strands, thereby determining the time course of the fluorescence intensity. The results are shown in Fig. 7.

### [Reference Example 1] Detection of sequence having a mutation by hybridization including annealing step

T2 and M2 were added to F2 (12 nM) each in an equal amount to that of F2, heated to 90°C and cooled slowly (annealing), and the fluorescence intensity was measured. The measurement was also made in the same manner, except that each of sequences having a mutation was used in place of M2. The relative values for the fluorescence intensities are shown in Table 6.

**Table 6: Ratio of fluorescence signal intensities after hybridization including annealing step**

| Mutation | |
|---|---|
| None (F2/M2) | 100 |
| T -> G (F2/M2misG) | 91.0 |
| T -> A (F2/M2misA) | 73.1 |
| T -> C (F2/M2misC) | 61.4 |

Even the sequences having a mutation showed fluorescence intensities of at least 60% relative to that of the entirely complementary strand. It is found that it is difficult to detect sequences having a single base mutation by the conventional hybridization including annealing step.

This specification includes part or all of the contents as disclosed in the specification and/or drawings of Japanese Patent Application No. 2001-253789, which is a priority document of the present application.

### INDUSTRIAL APPLICABILITY

The present invention provide a new method of judging whether or not a sequence mismatch occurs between a sample single-stranded nucleic acid molecule and a standard single-stranded nucleic acid molecule. The method makes it possible to discriminate the occurrence of a mismatch accurately even in the case where the mismatch occurs only in a single base. Accordingly, the method can be utilized in DNA diagnostics and the like.

## Claims

1. Use of a cationic polymer in a method for judging whether or not a sequence mismatch or mismatches occur between a sample single-stranded nucleic acid molecule and a standard single-stranded nucleic acid molecule wherein the method comprises:
(i)
(a) allowing a double-stranded nucleic acid molecule consisting of the standard single-stranded nucleic acid molecule and a complementary strand thereof to co-exist with the sample single-stranded nucleic acid molecule in the presence of a cationic polymer; and
(b) determining the rate or ratio of the substitution of the complementary strand of the standard single-stranded nucleic acid molecule by the sample single-stranded nucleic acid molecule; or
(ii)
(a) allowing the standard single-stranded nucleic acid molecule to contact with the sample single-stranded nucleic acid molecule to form a double-stranded nucleic acid molecule;
(b) allowing the double-stranded nucleic acid molecule thus formed to co-exist with an entirely complementary strand of the standard single-stranded nucleic acid molecule in the presence of a cationic polymer; and
(c) determining the rate or ratio of the substitution of the sample single-stranded nucleic acid molecule by the entirely complementary strand of the standard single-stranded nucleic acid molecule; or
(iii)
(a) allowing the standard single-stranded nucleic acid molecule to contact with an entirely complementary strand thereof and the sample single-stranded nucleic acid molecule in the presence of a cationic polymer; and
(b) determining the formation ratio between a double-stranded nucleic acid molecule formed from the standard single-stranded nucleic acid molecule and the entirely complementary strand thereof and a double-stranded nucleic acid molecule formed from the stranded nucleic acid molecule and the sample single-stranded nucleic acid molecule;

2. Use according to claim 1, wherein the cationic polymer is a graft copolymer having a main chain made up of a polymer composed of a monomer capable of forming a cationic group and a side chains made up of a hydrophilic polymer.

3. A method for judging whether or not a sequence mismatch or mismatches occur between a sample single-stranded nucleic acid molecule and a standard single-stranded nucleic acid molecule, the method comprising (a) allowing a double-stranded nucleic acid molecule consisting of the standard single-stranded nucleic acid molecule and a complementary strand thereof to co-exist with the sample single-stranded nucleic acid molecule in the presence of a cationic polymer; and (b) determining the rate or ratio of the substitution of the complementary strand of the standard single-stranded nucleic acid molecule by the sample single-stranded nucleic acid molecule.

4. A method for judging whether or not a sequence mismatch or mismatches occur between a sample single-stranded nucleic acid molecule and a standard single-stranded nucleic acid molecule, the method comprising (a) allowing the standard single-stranded nucleic acid molecule to contact with the sample single-stranded nucleic acid molecule to form a double-stranded nucleic acid molecule; (b) allowing the double-stranded nucleic acid molecule formed in (a) to co-exist with an entirely complementary strand of the standard single-stranded nucleic acid molecule in the presence of a cationic polymer; and (c) determining the rate or ratio of the substitution of the sample single-stranded nucleic acid molecule by the entirely complementary strand of the standard single-stranded nucleic acid molecule.

5. A method for judging whether or not a sequence mismatch or mismatches occur between a sample single-stranded nucleic acid molecule and a standard single-stranded nucleic acid molecule, the method comprising (a) allowing the standard single-stranded nucleic acid molecule to contact with an entirely complementary strand thereof and the sample single-stranded nucleic acid molecule in the presence of a cationic polymer; and (b) determining the formation ratio between a double-stranded nucleic acid molecule formed from the standard single-stranded nucleic acid molecule and the entirely complementary strand thereof and a double-stranded nucleic acid molecule formed from the standard single-stranded nucleic acid molecule and the sample single-stranded nucleic acid molecule.

6. The method for judging whether or not a mismatch or mismatches occur between the single-stranded nucleic acid molecules according to any one of claims 3 to 5, wherein the cationic polymer is a graft copolymer having a main chain made up of a polymer composed of a monomer capable of forming a cationic group and a side chains made up of a hydrophilic polymer.

7. The method for judging whether or not an mismatch or mismatches occur between the single-stranded nucleic acid molecules according to Claim 6, wherein the polymer composed of a monomer capable of forming a cationic group is polylysine or poly(allyl amine).

8. The method for judging whether or not a mismatch or mismatches occur between the single-stranded nucleic acid molecules according to claim 6 or 7, wherein the hydrophilic polymer is dextran or polyethylene glycol.

9. The method for judging whether or not a mismatch or mismatches occur between the single-stranded nucleic acid molecules according to any one of claims 3 to 5, wherein the cationic polymer is a polymer formed by the polymerization of a monomer having a phosphorylchlorine-like group represented by formula (I) and a cationic monomer having a cationic group: 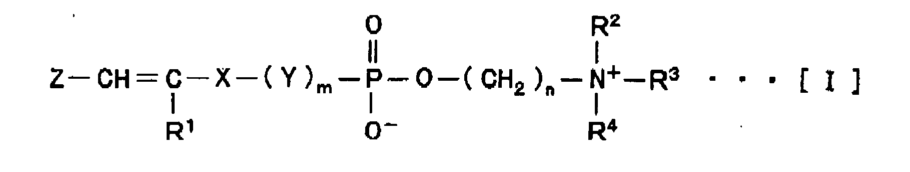 wherein X denotes a divalent organic residue; Y denotes an alkyleneoxy group having 1 to 6 carbon atoms; Z denotes a hydrogen atom or R⁵-O-(C=)) - wherein R⁵ denotes an alkyl group having 1 to 10 carbon atoms or a hydroxyalkyl group having 1 to 10 carbon atoms; R¹ denotes a hydrogen atom or a methyl group; each of R², R³ and R⁴ independently denotes a hydrogen atom or an alkyl or hydroxyalkyl group having 1 to 6 carbon atoms; m is 0 or 1; and n in an integer of 1 to 4.

## Patentansprüche

1. Verwendung eines kationischen Polymers in einem Verfahren zur Beurteilung, ob eine Sequenzfehlpaarung oder -fehlpaarungen zwischen einem als Probe dienenden einzelsträngigen Nukleinsäuremolekül und einem als Standard dienenden einzelsträngigen Nukleinsäuremolekül auftreten oder nicht, wobei das Verfahren umfasst:
(i)
(a) Ermöglichen, dass ein doppelsträngiges Nukleinsäuremolekül, das aus dem als Standard dienenden einzelsträngigen Nukleinsäuremolekül und einem dazu komplementären Strang besteht, mit dem als Probe dienenden einzelsträngigen Nukleinsäuremolekül nebeneinander in Gegenwart eines kationischen Polymers vorliegt; und
(b) Bestimmen der Geschwindigkeit oder des Verhältnisses der Substitution des komplementären Strangs des als Standard dienenden einzelsträngigen Nukleinsäuremoleküls durch das als Probe dienende einzelsträngige Nukleinsäuremolekül; oder
(ii)
(a) Ermöglichen, dass das als Standard dienende einzelsträngige Nukleinsäuremolekül mit dem als Probe dienenden einzelsträngigen Nukleinsäuremolekül in Kontakt kommt, um ein doppelsträngiges Nukleinsäuremolekül zu bilden;
(b) Ermöglichen, dass das so gebildete doppelsträngige Nukleinsäuremolekül mit einem völlig komplementären Strang des als Standard dienenden einzelsträngigen Nukleinsäuremoleküls nebeneinander in Gegenwart eines kationischen Polymers vorliegt; und
(c) Bestimmen der Geschwindigkeit oder des Verhältnisses der Substitution des als Probe dienenden einzelsträngigen Nukleinsäuremoleküls durch den völlig komplementären Strang des als Standard dienenden einzelsträngigen Nukleinsäuremoleküls; oder
(iii)
(a) Ermöglichen, dass das als Standard dienende einzelsträngige Nukleinsäuremolekül mit einem dazu völlig komplementären Strang und dem als Probe dienenden einzelsträngigen Nukleinsäuremolekül in Gegenwart eines kationischen Polymers in Kontakt kommt; und
(b) Bestimmen des Bildungsverhältnisses zwischen einem doppelsträngigen Nukleinsäuremolekül, das aus dem als Standard dienenden einzelsträngigen Nukleinsäuremolekül und dem dazu völlig komplementären Strang gebildet ist, und einem doppelsträngigen Nukleinsäuremolekül, das aus dem einzelsträngigen Nukleinsäuremolekül und dem als Probe dienenden einzelsträngigen Nukleinsäuremolekül gebildet ist.

2. Verwendung gemäß Anspruch 1, wobei das kationische Polymer ein Propfcopolymer ist, das eine Hauptkette aufweist, die aus einem Polymer aufgebaut ist, welches aus einem Monomer besteht, das in der Lage ist, eine kationische Gruppe zu bilden, und Seitenketten, die aus einem hydrophilen Polymer aufgebaut sind.

3. Verfahren zur Beurteilung, ob eine Sequenzfehlpaarung oder -fehlpaarungen zwischen einem als Probe dienenden einzelsträngigen Nukleinsäuremolekül und einem als Standard dienenden einzelsträngigen Nukleinsäuremolekül auftreten oder nicht, wobei das Verfahren umfasst (a) Ermöglichen, dass ein doppelsträngiges Nukleinsäuremolekül, das aus dem als Standard dienenden einzelsträngigen Nükleinsäuremolekül und einem dazu komplementären Strang besteht, mit dem als Probe dienenden einzelsträngigen Nukleinsäuremolekül nebeneinander in Gegenwart eines kationischen Polymers vorliegt; und (b) Bestimmen der Geschwindigkeit oder des Verhältnisses der Substitution des komplementären Strangs des als Standard dienenden einzelsträngigen Nukleinsäuremoleküls durch das als Probe dienende einzelsträngige Nukleinsäuremolekül.

4. Verfahren zur Beurteilung, ob eine Sequenzfehlpaarung oder -fehlpaarungen zwischen einem als Probe dienenden einzelsträngigen Nukleinsäuremolekül und einem als Standard dienenden einzelsträngigen Nukleinsäuremolekül auftreten oder nicht, wobei das Verfahren umfasst (a) Ermöglichen, dass das als Standard dienende einzelsträngige Nukleinsäuremolekül mit dem als Probe dienenden einzelsträngigen Nukleinsäuremolekül in Kontakt kommt, um ein doppelsträngiges Nukleinsäuremolekül zu bilden; (b) Ermöglichen, dass das in (a) gebildete doppelsträngige Nukleinsäuremolekül mit einem völlig komplementären Strang des als Standard dienenden einzelsträngigen Nukleinsäuremoleküls nebeneinander in Gegenwart eines kationischen Polymers vorliegt; und (c) Bestimmen der Geschwindigkeit oder des Verhältnisses der Substitution des als Probe dienenden einzelsträngigen Nukleinsäuremoleküls durch den völlig komplementären Strang des als Standard dienenden einzelsträngigen Nukleinsäuremoleküls.

5. Verfahren zur Beurteilung, ob eine Sequenzfehlpaarung oder -fehlpaarungen zwischen einem als Probe dienenden einzelsträngigen Nukleinsäuremolekül und einem als Standard dienenden einzelsträngigen Nukleinsäuremolekül auftreten oder nicht, wobei das Verfahren umfasst (a) Ermöglichen, dass das als Standard dienende einzelsträngige Nukleinsäuremolekül mit einem dazu völlig komplementären Strang und dem als Probe dienenden einzelsträngigen Nukleinsäuremolekül in Gegenwart eines kationischen Polymers in Kontakt kommt; und (b) Bestimmen des Bildungsverhältnisses zwischen einem doppelsträngigen Nukleinsäuremolekül, das aus dem als Standard dienenden einzelsträngigen Nukleinsäuremolekül und dem dazu völlig komplementären Strang gebildet ist, und einem doppelsträngigen Nukleinsäuremolekül, das aus dem als Standard dienenden einzelsträngigen Nukleinsäuremolekül und dem als Probe dienenden einzelsträngigen Nukleinsäuremolekül gebildet ist.

6. Verfahren zur Beurteilung, ob eine Fehlpaarung oder Fehlpaarungen zwischen den einzelsträngigen Nukleinsäuremolekülen auftreten oder nicht gemäß einem der Ansprüche 3 bis 5, wobei das kationische Polymer ein Propfcopolymer ist, das eine Hauptkette aufweist, die aus einem Polymer aufgebaut ist, welches aus einem Monomer besteht, das in der Lage ist, eine kationische Gruppe zu bilden, und Seitenketten, die aus einem hydrophilen Polymer aufgebaut sind.

7. Verfahren zur Beurteilung, ob eine Fehlpaarung oder Fehlpaarungen zwischen den einzelsträngigen Nukleinsäuremolekülen auftreten oder nicht gemäß Anspruch 6, wobei das Polymer, welches aus einem Monomer besteht, das in der Lage ist, eine kationische Gruppe zu bilden, Polylysin oder Poly(allylamin) ist.

8. Verfahren zur Beurteilung, ob eine Fehlpaarung oder Fehlpaarungen zwischen den einzelsträngigen Nukleinsäuremolekülen auftreten oder nicht gemäß Anspruch 6 oder 7, wobei das hydrophile Polymer Dextran oder Polyethylenglykol ist.

9. Verfahren zur Beurteilung, ob eine Fehlpaarung oder Fehlpaarungen zwischen den einzelsträngigen Nukleinsäuremolekülen auftreten oder nicht gemäß einem der Ansprüche 3 bis 5, wobei das kationische Polymer ein Polymer ist, das durch die Polymerisation eines Monomers, welches eine Phosphorylchlor-artige Gruppe aufweist, die durch Formel (I) dargestellt ist, und eines kationischen Monomers, das eine kationische Gruppe aufweist, gebildet ist:

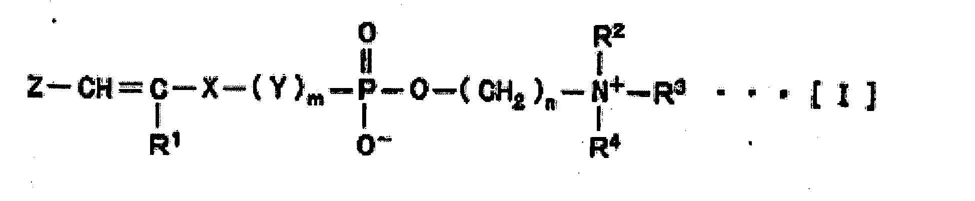

## Revendications

1. Utilisation d'un polymère cationique dans un procédé pour évaluer si un ou plusieurs mésappariements entre des séquences apparaît/apparaissent entre une molécule d'acide nucléique monobrin échantillon et une molécule d'acide nucléique monobrin standard, dans laquelle le procédé comprend les étapes suivantes :
(i)
(a) autoriser une molécule d'acide nucléique double brin constituée de la molécule d'acide nucléique monobrin standard et d'un brin complémentaire de celle-ci à coexister avec la molécule d'acide nucléique monobrin échantillon en présence d'un polymère cationique ; et
(b) déterminer la vitesse ou le taux de substitution du brin complémentaire de la molécule d'acide nucléique monobrin standard par la molécule d'acide nucléique monobrin échantillon ; ou
(ii)
(a) autoriser la molécule d'acide nucléique monobrin standard à venir au contact de la molécule d'acide nucléique monobrin échantillon pour former une molécule d'acide nucléique double brin ;
(b) autoriser la molécule d'acide nucléique double brin ainsi formée à coexister avec un brin entièrement complémentaire de la molécule d'acide nucléique monobrin standard en présence d'un polymère cationique ; et
(c) déterminer la vitesse ou le taux de substitution de la molécule d'acide nucléique monobrin échantillon par le brin entièrement complémentaire de la molécule d'acide nucléique monobrin standard ; ou
(iii)
(a) autoriser la molécule d'acide nucléique monobrin standard à venir au contact d'un brin entièrement complémentaire de celle-ci et de la molécule d'acide nucléique monobrin échantillon en présence d'un polymère cationique ; et
(b) déterminer le taux de formation entre une molécule d'acide nucléique double brin formée de la molécule d'acide nucléique monobrin standard et du brin entièrement complémentaire de celle-ci et d'une molécule d'acide nucléique double brin formée de la molécule d'acide nucléique monobrin et de la molécule d'acide nucléique monobrin échantillon ;

2. Utilisation selon la revendication 1, dans laquelle le polymère cationique est un copolymère greffé ayant une chaîne principale faite d'un polymère composé d'un monomère capable de former un groupe cationique et de chaînes latérales faites d'un polymère hydrophile.

3. Procédé pour évaluer si un ou plusieurs mésappariements entre des séquences apparaît/apparaissent entre une molécule d'acide nucléique monobrin échantillon et une molécule d'acide nucléique monobrin standard, le procédé comprenant (a) autoriser une molécule d'acide nucléique double brin constituée de la molécule d'acide nucléique monobrin standard et d'un brin complémentaire de celle-ci à coexister avec la molécule d'acide nucléique monobrin échantillon en présence d'un polymère cationique ; et (b) déterminer la vitesse ou le taux de substitution du brin complémentaire de la molécule d'acide nucléique monobrin standard par la molécule d'acide nucléique monobrin dans l'échantillon.

4. Procédé pour évaluer si un ou plusieurs mésappariements entre des séquences apparaît/apparaissent entre une molécule d'acide nucléique monobrin échantillon et une molécule d'acide nucléique monobrin standard, le procédé comprenant (a) autoriser la molécule d'acide nucléique monobrin standard à venir au contact de la molécule d'acide nucléique monobrin échantillon pour former une molécule d'acide nucléique double brin ; (b) autoriser la molécule d'acide nucléique double brin formée en (a) à coexister avec un brin entièrement complémentaire de la molécule d'acide nucléique monobrin standard en présence d'un polymère cationique ; et (c) déterminer la vitesse ou le taux de substitution de la molécule d'acide nucléique monobrin échantillon par le brin entièrement complémentaire de la molécule d'acide nucléique monobrin standard.

5. Procédé pour évaluer si un ou plusieurs mésappariements entre des séquences apparaît/apparaissent entre une molécule d'acide nucléique monobrin échantillon et une molécule d'acide nucléique monobrin standard, le procédé comprenant (a) autoriser la molécule d'acide nucléique monobrin standard à venir au contact d'un brin entièrement complémentaire de celle-ci et de la molécule d'acide nucléique monobrin échantillon en présence d'un polymère cationique ; et (b) déterminer le taux de formation entre une molécule d'acide nucléique double brin formée de la molécule d'acide nucléique monobrin standard et du brin entièrement complémentaire de celle-ci et d'une molécule d'acide nucléique double brin formée de la molécule d'acide nucléique monobrin standard et de la molécule d'acide nucléique monobrin échantillon.

6. Procédé pour évaluer si un ou plusieurs mésappariements apparaît/apparaissent entre les molécules d'acides nucléiques monobrin selon l'une quelconque des revendications 3 à 5, dans lequel le polymère cationique est un copolymère greffé ayant une chaîne principale faite d'un polymère composé d'un monomère capable de former un groupe cationique et de chaînes latérales faites d'un polymère hydrophile.

7. Procédé pour évaluer si un ou plusieurs mésappariements apparaît/apparaissent entre les molécules d'acides nucléiques monobrin selon la revendication 6, dans lequel le polymère composé d'un monomère capable de former un groupe cationique est de la polylysine ou de la poly(allylamine).

8. Procédé pour évaluer si un ou plusieurs mésappariements apparaît/apparaissent entre les molécules d'acide nucléique monobrin selon la revendication 6 ou 7, dans lequel le polymère hydrophile est du dextrane ou du polyéthylèneglycol.

9. Procédé pour évaluer si un ou plusieurs mésappariements apparaît/apparaissent entre les molécules d'acides nucléiques monobrin selon l'une quelconque des revendications 3 à 5, dans lequel le polymère cationique est un polymère formé par polymérisation d'un monomère ayant un groupe ressemblant à de la phosphorylchlorine, représenté par la formule (I), et d'un monomère cationique ayant un groupe cationique : 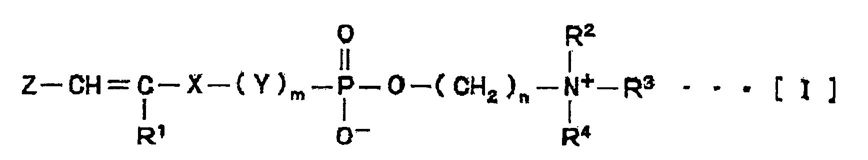 où X signifie un résidu organique divalent ; Y signifie un groupe alkylèneoxy ayant de 1 à 6 atomes de carbone ; Z signifie un atome d'hydrogène ou R⁵-O- (C=) ) - où R⁵ signifie un groupe alkyle ayant de 1 à 10 atomes de carbone ou un groupe hydroxyalkyle ayant de 1 à 10 atomes de carbone ; R¹ signifie un atome d'hydrogène ou un groupe méthyle ; R², R³ et R⁴ signifient chacun indépendamment un atome d'hydrogène ou un groupe alkyle ou hydroxyalkyle ayant de 1 à 6 atomes de carbone ; m vaut 0 ou 1 ; et n est un nombre entier de 1 à 4.


### SEQUENCE LISTING

<110> RIKOHGAKUSHINKOHKAI
<120> METHOD FOR DETECTING MISMATCH BETWEEN SINGLE NUCLEIC ACID MOLECULES
<130> FP-013PCT
<150> JP 2001-253789
   <151> 2001-08-24
<160> 17
<170> PatentIn Ver. 2.1
<210> 1
   <211> 20
   <212> DNA
   <213> Artificial Sequence
<400>
   atggtgagca agggcgagga 20
<210> 2
   <211> 20
   <212> DNA
   <213> Artificial Sequence
<400> 2
   tcctcgccct tgctcaccat 20
<210> 3
   <211> 20
   <212> DNA
   <213> Artificial Sequence
<400> 3
   tcctcgccca tgctcaccat 20
<210> 4
   <211> 20
   <212> DNA
   <213> Artificial Sequence
<400> 4
   tcctcgcccg tgctcaccat 20
<210> 5
   <211> 20
   <212> DNA
   <213> Artificial Sequence
<400> 5
   tcctcgcccc tgctcaccat 20
<210> 6
   <211> 20
   <212> DNA
   <213> Artificial Sequence
<400> 6
   tccgcgccct tgctcaccat 20
<210> 7
   <211> 20
   <212> DNA
   <213> Artificial Sequence
<400> 7
   gcctcgccct tgctcaccat 20
<210> 8
   <211> 20
   <212> DNA
   <213> Artificial Sequence
<400> 8
   tcataatcag ccataccaca 20
<210> 9
   <211> 20
   <212> DNA
   <213> Artificial Sequence
<400> 9
   tgtggtatgg ctgattatga 20
<210> 10
   <211> 20
   <212> DNA
   <213> Artificial Sequence
<400> 10
   tgtggtatga ctgattatga 20
<210> 11
   <211> 20
   <212> DNA
   <213> Artificial Sequence
<400> 11
   tgtggtatgt ctgattatga 20
<210> 12
   <211> 20
   <212> DNA
   <213> Artificial Sequence
<400> 12
   tgtggtatgc ctgattatga 20
<210> 13
   <211> 50
   <212> DNA
   <213> Artificial Sequence
<400> 13
   accccggtga acagctcctc gcccttgctc accatggtgg cgaccggccg 50
<210> 14
   <211> 50
   <212> DNA
   <213> Artificial Sequence
<400> 14
   accccggtga acagctcctc gcccgtgctc accatggtgg cgaccggccg 50
<210> 15
   <211> 19
   <212> DNA
   <213> Artificial Sequence
<400> 15
   ttatttccca ggaacccat 19
<210> 16
   <211> 19
   <212> DNA
   <213> Artificial Sequence
<400> 16
   atgggttcct gggaaataa 19
<210> 17
   <211> 19
   <212> DNA
   <213> Artificial Sequence
<400> 17
   atgggttccc gggaaataa 19

### SEQUENCE LISTING

<110> RIKOHGAKUSHINKOHKAI
<120> METHOD FOR DETECTING MISMATCH BETWEEN SINGLE NUCLEIC ACID MOLECULES
<130> FP-013PCT
<150> JP 2001-253789
   <151> 2001-08-24
<160> 17
<170> PatentIn Ver. 2. 1
<210> 1
   <211> 20
   <212> DNA
   <213> Artificial Sequence
<400> 1
   atggtgagca agggcgagga 20
<210> 2
   <211> 20
   <212> DNA
   <213> Artificial Sequence
<400> 2
   tcctcgccct tgctcaccat 20
<210> 3
   <211> 20
   <212> DNA
   <213> Artificial Sequence
<400> 3
   tcctcgccca tgctcaccat 20
<210> 4
   <2H> 20
   <212> DNA
   <213> Artificial Sequence
<400> 4
   tcctcgcccg tgctcaccat 20
<210> 5
   <211> 20
   <212> DNA
   <213> Artificial Sequence
<400> 5
   tcctcgcccc tgctcaccat 20
<210> 6
   <211> 20
   <212> DNA
   <213> Artificial Sequence
<400> 6
   tccgcgccct tgctcaccat 20
<210> 7
   <211> 20
   <212> DNA
   <213> Artificial Sequence
<400> 7
   gcctcgccct tgctcaccat 20
<210> 8
   <211> 20
   <212> DNA
   <213> Artificial Sequence
<400> 8
   tcataatcag ccataccaca 20
<210> 9
   <211> 20
   <212> DNA
   <213> Artificial Sequence
<400> 9
   tgtggtatgg ctgattatga 20
<210> 10
   <211> 20
   <212> DNA
   <213> Artificial Sequence
<400> 10
   tgtggtatga ctgattatga 20
<210> 11
   <211> 20
   <212> DNA
   <213> Artificial Sequence
<400> 11
   tgtggtatgt ctgattatga 20
<210> 12
   <211> 20
   <212> DNA
   <213> Artificial Sequence
<400> 12
   tgtggtatgc ctgattatga 20
<210> 13
   <211> 50
   <212> DNA
   <213> Artificial Sequence
<400> 13
   accccggtga acagctcctc gcccttgctc accatggtgg cgaccggccg 50
<210> 14
   <211> 50
   <212> DNA
   <213> Artificial Sequence
<400> 14
   accccggtga acagctcctc gcccgtgctc accatggtgg cgaccggccg 50
<210> 15
   <211> 19
   <212> DNA
   <213> Artificial Sequence
<400> 15
   ttatttccca ggaacccat 19
<210> 16
   <211> 19
   <212> DNA
   <213> Artificial Sequence
<400> 16
   atgggttcct gggaaataa 19
<210> 17
   <211> 19
   <212> DNA
   <213> Artificial Sequence
<400> 17
   atgggttccc gggaaataa 19

### SEQUENCE LISTING

<110> NOF Corporation
<120> METHOD FOR DETECTING MISMATCH BETWEEN SINGLE NUCLEIC ACID MOLECULES
<130> N90832 GCW
<140> EP 02762825.4
   <141> 2001-08-24
<150> JP 2001-253789
   <151> 2001-08-24
<160> 17
<170> Patent In Ver. 2.1
<210> 1
   <211> 20
   <212> DNA
   <213> Artificial Sequence
<220>
   <221> source
   <223> oligonucletide hybridisation probe
<400> 1
   atggtgagca agggcgagga 20
<210> 2
   <211> 20
   <212> DNA
   <213> Artificial Sequence
<220>
   <221> source
   <223> oligonucletide hybridisation probe
<400> 2
   tcctcgccct tgctcaccat 20
<210> 3
   <211> 20
   <212> DNA
   <213> Artificial Sequence
<220>
   <221> source
   <223> oligonucletide hybridisation probe
<400> 3
   tcctcgccca tgctcaccat 20
<210> 4
   <211> 20
   <212> DNA
   <213> Artificial Sequence
<220>
   <221> source
   <223> oligonucletide hybridisation probe
<400> 4
   tcctcgcccg tgctcaccat 20
<210> 5
   <211> 20
   <212> DNA
   <213> Artificial Sequence
<220>
   <221> source
   <223> oligonucletide hybridisation probe
<400> 5
   tcctcgcccc tgctcaccat 20
<210> 6
   <211> 20
   <212> DNA
   <213> Artificial Sequence
<220>
   <221> source
   <223> oligonucletide hybridisation probe
<400> 6
   tccgcgccct tgctcaccat 20
<210> 7
   <211> 20
   <212> DNA
   <213> Artificial Sequence
<220>
   <221> source
   <223> oligonucletide hybridisation probe
<400> 7
   gcctcgccct tgctcaccat 20
<210> 8
   <211> 20
   <212> DNA
   <213> Artificial Sequence
<220>
   <221> source
   <223> oligonucletide hybridisation probe
<400> 8
   tcataatcag ccataccaca 20
<210> 9
   <211> 20
   <212> DNA
   <213> Artificial Sequence
<220>
   <221> source
   <223> oligonucletide hybridisation probe
<400> 9
   tgtggtatgg ctgattatga 20
<210> 10
   <211> 20
   <212> DNA
   <213> Artificial Sequence
<220>
   <221> source
   <223> oligonucletide hybridisation probe
<400> 10
   tgtggtatga ctgattatga 20
<210> 11
   <211> 20
   <212> DNA
   <213> Artificial Sequence
<220>
   <221> source
   <223> oligonucletide hybridisation probe
<400> 11
   tgtggtatgt ctgattatga 20
<210> 12
   <211> 20
   <212> DNA
   <213> Artificial Sequence
<220>
   <221> source
   <223> oligonucletide hybridisation probe
<400> 12
   tgtggtatgc ctgattatga 20
<210> 13
   <211> 50
   <212> DNA
   <213> Artificial Sequence
<220>
   <221> source
   <223> oligonucletide hybridisation probe
<400> 13
   accccggtga acagctcctc gcccttgctc accatggtgg cgaccggccg 50
<210> 14
   <211> 50
   <212> DNA
   <213> Artificial Sequence
<220>
   <221> source
   <223> oligonucletide hybridisation probe
<400> 14
   accccggtga acagctcctc gcccgtgctc accatggtgg cgaccggccg 50
<210> 15
   <211> 19
   <212> DNA
   <213> Artificial Sequence
<220>
   <221> source
   <223> oligonucletide hybridisation probe
<400> 15
   ttatttccca ggaacccat 19
<210> 16
   <211> 19
   <212> DNA
   <213> Artificial Sequence
<220>
   <221> source
   <223> oligonucletide hybridisation probe
<400> 16
   atgggttcct gggaaataa 19
<210> 17
   <211> 19
   <212> DNA
   <213> Artificial Sequence
<220>
   <221> source
   <223> oligonucletide hybridisation probe
<400> 17
   atgggttccc gggaaataa 19